# EUROPEAN PATENT SPECIFICATION

(11) **EP 3 558 433 B1**
(45) Date of publication and mention of the grant of the patent: **01.09.2021**
(21) Application number: 17817177.3
(22) Date of filing: 04.12.2017
(51) Int. Cl.: A61M 16/06

(54) **INFLATABLE FRAMELESS MASK FOR THE VENTILATION OF PATIENTS**
AUFBLASBARE, RAHMENLOSE MASKE ZUR BEATMUNG VON PATIENTEN
MASQUE GONFLABLE SANS CADRE POUR LA VENTILATION DE PATIENTS

(30) Priority: 22.12.2016 IT 201600129798
(43) Date of publication of application: 30.10.2019
(73) Proprietor: Intersurgical S.P.A., 41037 Mirandola, Modena (IT)
(72) Inventor: ROSSI, Paolo, 41037 Mirandola (MO) (IT)
(74) Representative: Corradini, Corrado
(86) International application number: PCT/IB2017/057603
(87) International publication number: WO 2018/116040

(56) References cited:
- EP-A1- 2 684 578
- WO-A2-2007/030783
- US-A- 4 231 359
- US-A- 5 819 728
- US-A1- 2016 271 355

## Description

### TECHNICAL FIELD

The present invention relates to a frameless mask for the forced ventilation of patients and the method for the manufacture of a frameless full-face mask.

More specifically, the invention relates to a mask, e.g. a full-face, frameless mask for CPAP or NIV therapy.

### PRIOR ART

For the CPAP or NIV therapy, known masks are used which can be included within three broad categories.

A first category is that of nasal masks, i.e. those that surround only the patient's nose, a second category is that of oro-nasal masks, i.e. those that surround the patient's nose and mouth and a third category is that of full-face masks, i.e. masks that surround the patient's entire face, i.e. his/her eyes, nose and mouth.

Full-face masks generally consist of a semi-rigid shell, e.g. obtained by molding, which has an annular peripheral edge intended to go in contact, with the interposition of a gasket e.g. made of silicone, with the patient's face, i.e. with the perimeter thereof which inscribes mouth, nose and eyes.

The semi-rigid shell is usually optically transparent and has substantially undeformable shape and a weight that can be, perceived by the user as very significant, especially for long-term uses.

An object of the present invention is to overcome the aforementioned drawbacks of the prior art, within the scope of a solution that is simple, rational, comfortable for the user, light and low-cost.

These objects are achieved by the characteristics of the invention set out in the independent claim. The dependent claims outline preferred and/or particularly advantageous aspects of the invention. The following documents disclose known devices for the ventilation of a patient: EP 2 684 578 US 4 231 359, US 5 819 728, WO 2007/030783.

### DISCLOSURE OF THE INVENTION

The invention is defined in the appended claims. The invention, in particular, makes available an inflatable frameless mask tor the ventilation of patients comprising:
- a flexible and inextensible shell provided with a peripheral edge generally closed in a loop able to come in forced, fluid-tight contact with the face of the patient and to surround at least the nose of the patient,
- inflow means for injecting a breathable gas positioned on the shell for the injection of breathable gas inside a volume enclosed between the shell and the face of the patient;
- inflow means for the outflow of gases exhaled by the patient for the exit from the volume of the gases exhaled by the patient;
- wherein the breathable gas introduced into the closed volume by the injection means inflates the shell from a deflated configuration of the shell to an inflated configuration of the shell, wherein in the inflated configuration the breathable gas inside the closed volume is at a higher pressure than ambient pressure; and
- wherein at least one portion of the flexible shell is optically transparent,
- wherein the mask (10) comprises a nuchal strap (90) associated with the shell (20) for anchoring the mask (10) to the patient's head.

Thanks to this solution it is possible to make available a light, economical and functional mask, which at the same time enables the complete monitoring of the user's airways, including visually.

According to one aspect of the invention, the peripheral edge can support an annular gasket, for example an inflatable one.

Thanks to this solution, although the mask is already flexible, it can increase its seal on the patient's face with reduced contact pressure (due to the large contact surface area) on the patient's face, which entails better comfort for the patient.

For example, the mask can comprise an inflation valve on the annular gasket Thanks to this solution, it is possible to change at will the inflation pressure of the annular gasket and the configuration (and size) of the annular gasket.

Advantageously, the shell can be completely optically transparent.

Preferably, the shell of made of a single layer of gas-impermeable plastic material.

Thanks to this solution the mask is particularly light, comfortable and effective.

According to the invention, the mask comprises a nuchal strap associated with the shell for anchoring the mask to the patient's head. The shell can also comprise a flat face joined to a peripheral liner closed substantially in a loop, whose first axial end is sealingly joined to the perimeter of the flat face and whose second axial end is free and defines the peripheral edge of the shell.

Thanks to this solution, fabrication of the mask can be simplified and production costs can be reduced.

Preferably, but without limitation, the mask can be a full-face mask and the edge is able to surround the patient's mouth, nose and eyes.

The invention also makes available a method for the manufacture of the above inflatable frameless mask for the ventilation of patients comprising the following steps:
- shaping as a flexible and inextensible shell provided with a peripheral edge generally closed in a loop able to come in forced, fluid-tight contact with the face of the patient and to surround at least the nose of the patient, wherein the shaping step comprises the step of
   - joining a first shaped portion of a film of a flexible and inextensible plastic material to a second portion of a film of a flexible and inextensible plastic material by radio frequency welding or heat sealing,
- obtaining, by cutting a layer of fabric or nonwoven fabric or another material, a nuchal strap (90) for the completion of the mask (10),
- wherein at least one between the first shaped portion and the second shaped portion is at least partially optically transparent.

Thanks to this solution it is possible to obtain a mask in an economical and simple manner with high productivity and with advantages in terms of weight and comfort of the mask directly obtained.

Advantageously, the method can comprise the step of joining to the peripheral edge of the shell an annular gasket by radio frequency welding or heat sealing.

According to the invention, at least one between the first shaped portion and the second shaped portion is at least partially optically transparent. Advantageously, the first shaped portion can be conformed as a flat face and the second shaped portion can be conformed as a peripheral liner closed substantially as a loop, whose first axial end is sealingly joined, by radio frequency welding or heat sealing, to the entire perimeter of the flat face and whose second axial end is free and defines the peripheral edge of the shell.

Thanks to this solution the industrialization of the process for producing the mask appears decidedly simplified with respect to the processes for producing known masks.

### BRIEF DESCRIPTION OF THE DRAWINGS

Additional characteristics and advantages of the invention shall become readily apparent from the description that follows, provided by way of example but without limitation, with the aid of the figures illustrated in the accompanying table.
Figure 1 is an axonometric view of a mask according to the invention, in an inflated configuration and to which is schematically fastened a nuchal strap.
Figure 2 is an axonometric view of the mask of figure 1, in which the nuchal is not shown, in the inflated configuration.
Figure 3 is a bottom view (according to the view III of figure 2) of figure 2.
Figure 4 is a front view of figure 2.
Figure 5 is a section view along the section V-V of figure 4 of an anti-suffocation valve.
Figure 6 is a lateral view (from the left) of figure 2.
Figure 7 is a lateral view (from the left) of the mask of figure 2 in a deflated configuration.

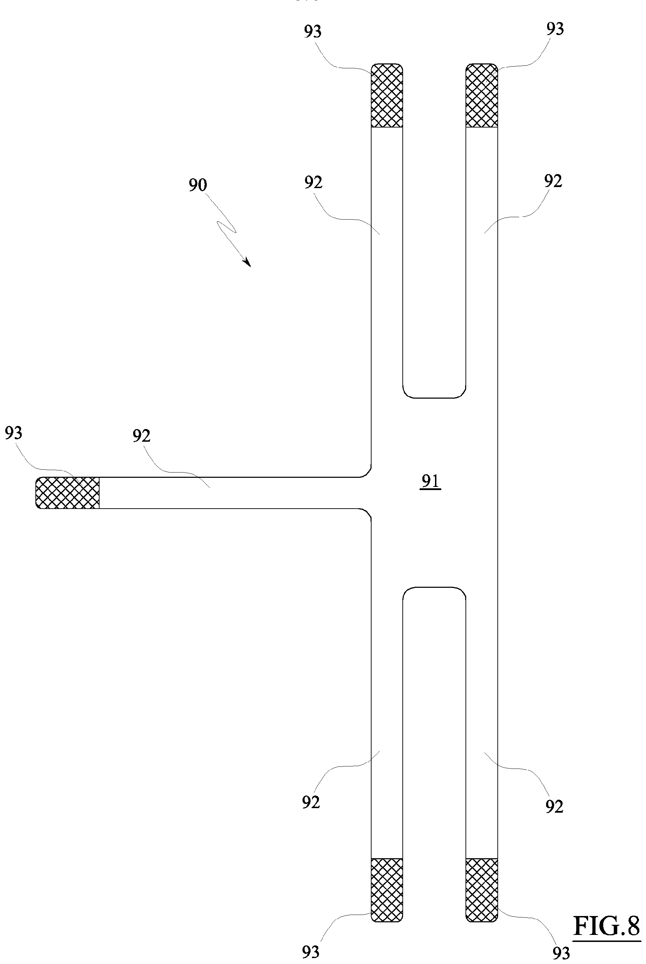

Figure 8 is a plan view of a nuchal strap according to the invention.

### BEST EMBODIMENT OF THE INVENTION

With particular reference to the figures, the reference number 10 indicates in its entirety a mask, e.g. a full-face mask, the term "full-face mask" meaning a mask able to surround the nose, mouth and eyes of a user, i.e. a patient.

The mask 10 may nonetheless be of the nasal type, i.e. able to surround only the nose of the patient, or of the oro-nasal type, i.e. able to surround only the nose and mouth of the patient.

The mask 10 is for example generally frameless, i.e. without rigid structural parts that determine as a whole a predefined undeformable or substantially undeformable shape thereof.

In practice, the mask 10 can be bent, creased without thereby compromising its structural and functional integrity, as shall be described better hereafter.

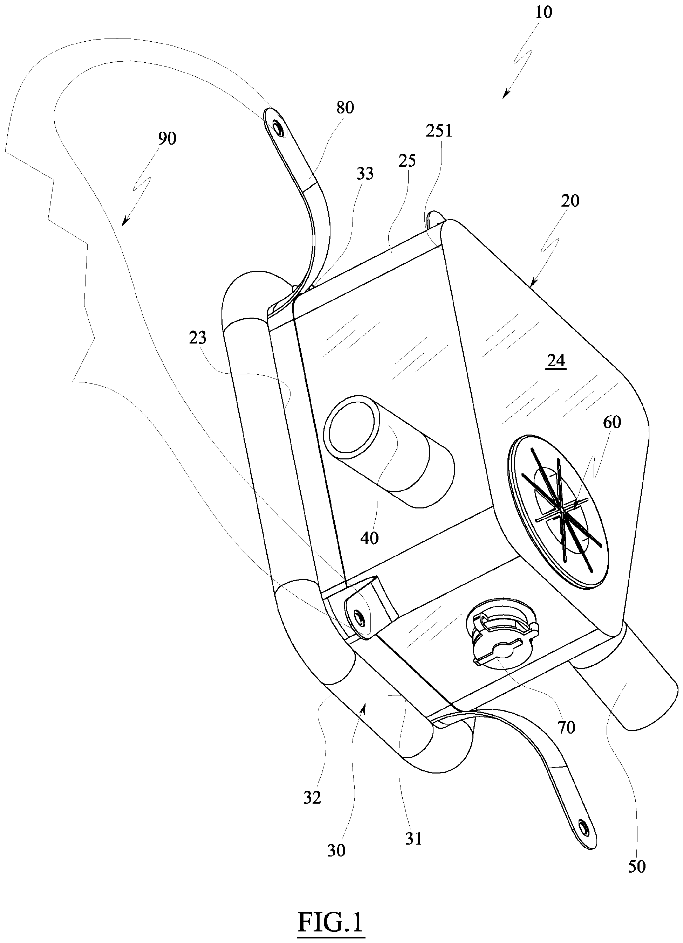

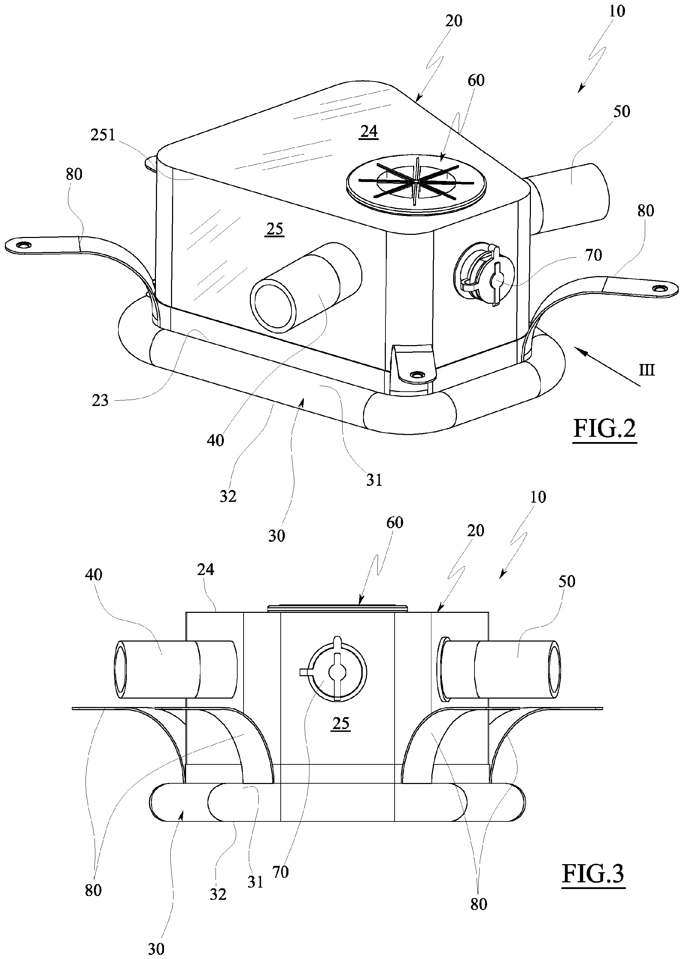

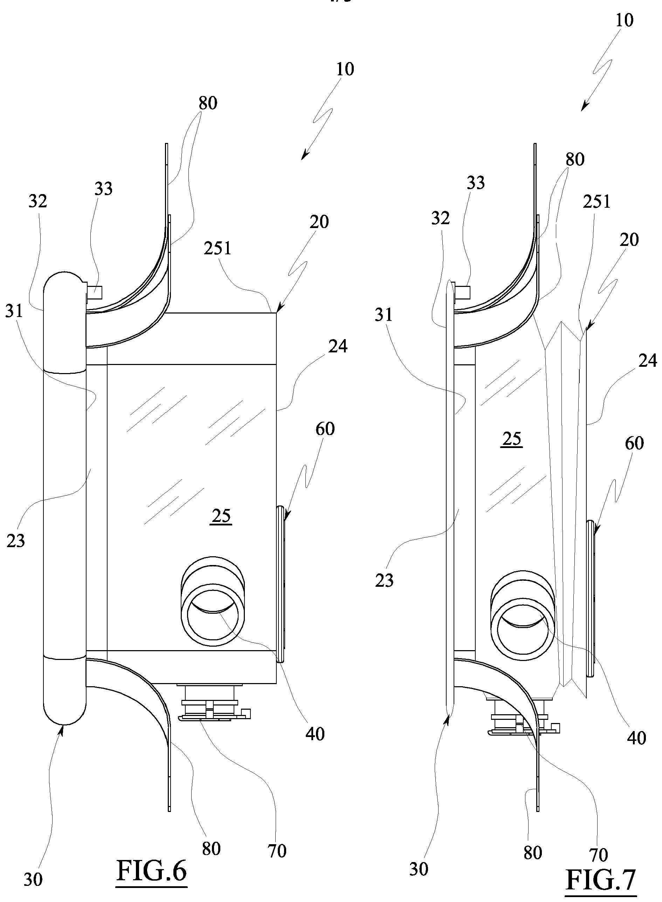

The mask 10 as a whole is for example an inflatable mask, the term "inflatable" meaning that it can alternatively shift between a first deflated configuration (figure 7) and a (different) inflated configuration (figures 1-6), wherein in the inflated configuration the volume of the mask 10 is different, preferably greater, than the volume of the mask 10 in the deflated configuration.

For example, the mask 10 as a whole is able to shift from the deflated configuration to the inflated configuration by effect of the operating pressure, higher than atmospheric pressure, at which a breathable gas is supplied to be made available for inhalation by a user of the mask 10, as shall become more readily apparent hereafter.

The mask 10 comprises a shell 20, which shell 20 is for example substantially concave, with concavity oriented towards the patient's face.

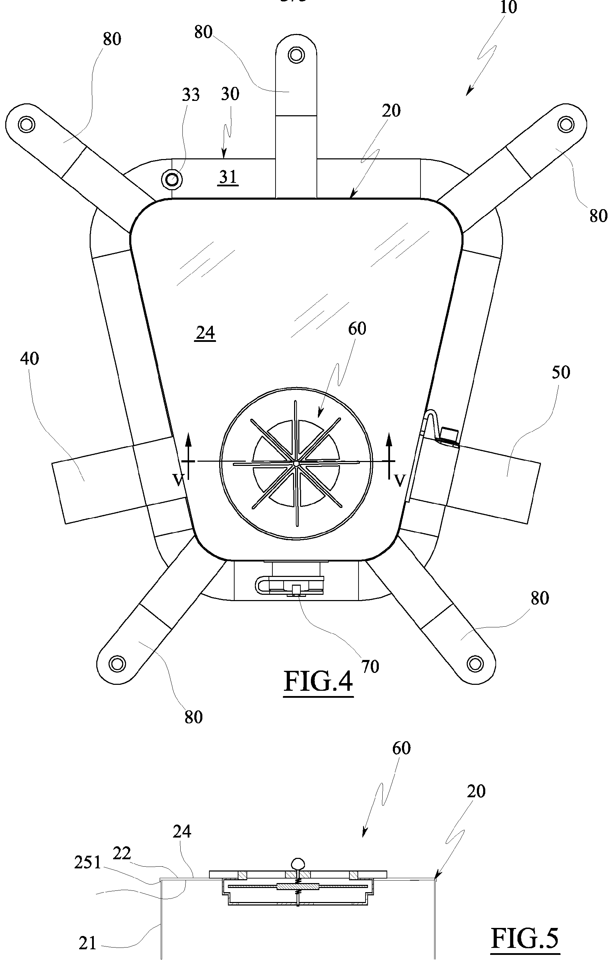

The shell 20 thus has a concave inner side 21 (figure 5) and an opposite convex outer side.

The shell 20 as a whole is made of a flexible and inextensible material, e.g. with reduced (if not nil) tensile elasticity.

The shell 20 is made, for example, of Polyvinylchloride (PVC) or another material.

For example, the shell 20 comprises a peripheral edge 23 closed substantially in a loop, e.g. shaped, which is able to go in contact (as shall be described better hereafter) with a frontal line of the patient's face that surrounds his/her mouth, nose and eyes.

The edge 23 has substantially trapezoidal shape (with rounded corners), e.g. of an isosceles trapezoid, with the shorter base positioned below in operation (in the chin region) and the longer base positioned above (in the forehead region).

In practice, a shorter lower base of the edge 23 is able to rest (though not directly) on the patient's chin (below the mouth), a longer upper base of the edge 23 is able to rest (though not directly) on the patient's forehead (above the eyes) and two sides that join the longer upper base to the shorter lower base are able to rest (though not directly) on the side of the face, i.e. the eyes and cheek contour (to the side of the eyes, of the nose and of the mouth).

The trapezoidal shape can be, for example, also substantially triangular or ovalized or circular depending on requirements.

The shell 20, when the edge 23 rests on the patient's face, delimits a closed volume communicating, through the patient's mouth and nose, with the respiratory system of the patient.

The shell 20 is for example formed by one or more single-layer sheets of gas-impermeable material, e.g. a plastic material, e.g. not (completely) superposed on each other.

In practice, each single-layer sheet that forms the shell 20 defines at least one portion of the inner side 21 of the shell 20 and at least one portion of the outer side 22 of the shell 20.

The shell 20 is, for example, formed by a flat face 24 (or visor), which is at least as broad as the portion of the face surrounded by the edge 23 of the shell 20.

The flat face 24 has substantially trapezoidal shape (with rounded corners), e.g. of an isosceles trapezoid, with the shorter base oriented downwards and the longer base oriented upwards.

To the outer perimeter (peripheral end) of the flat face 24 is joined a peripheral liner 25 (generally prismatic, e.g. with homologous section to the shape of the flat face, i.e. trapezoidal) closed substantially in a loop that rises substantially orthogonal to the flat face 24.

The cross section of the peripheral liner 25 has homologous shape to the perimeter of the flat face 24 (e.g. constant along its entire axial development, i.e. along the longitudinal axis of the peripheral liner 25, or the orthogonal axis to the flat face 24).

A first axial end 251 of the peripheral liner 25 is joined (totally and seamlessly) sealingly to the outer perimeter of the flat face 24, and a second axial end of the peripheral liner 25 is free and defines *de facto* the edge 23 of the shell 20.

The shell 20 thus substantially consists of the peripheral liner 25 and of the flat face 24.

The flat face 24 consists for example of a single-layer sheet of gas-impermeable material, e.g. a plastic material.

For example, the flat face is made of a flexible and inextensible material.

For example, the flat face 24 is between 0.2 mm and 0.5 mm thick, preferably 0.3 mm.

The flat face 24 is, for example, made of PVC or another material.

In practice, the flat face 24 has an outer face oriented towards the opposite part of the peripheral liner 25, which outer face defines a portion of the outer side 22 of the shell 20, and an opposite inner face oriented towards the interior of the peripheral liner 25, which inner face defines a portion of the inner side 21 of the shell 20.

The peripheral liner 25 also consists for example of a single-layer sheet of gas-impermeable material, e.g. a plastic material.

For example, the peripheral liner 25 is made of a flexible and inextensible material.

For example, the peripheral liner 25 is between 0.2 mm and 0.5 mm thick, preferably 0.3 mm.

The peripheral liner 25 is, for example, made of PVC or another material.

In practice, the peripheral liner 25 has a concave outer face (oriented towards the outer face of the peripheral liner), which outer face defines a portion of the outer side 22 of the shell 20, and an opposite convex inner face (oriented towards the inner face of the peripheral liner 25), which inner face defines a portion of the inner side 21 of the shell 20.

In practice, the inner side 21 of the shell 20 consists of the inner face of the peripheral liner 25 and of the inner face of the flat face 24 and the outer side 22 of the shell 20 consists of the outer face of the peripheral liner 25 and of the outer face of the flat face 24.

The shell 20 is inflatable as a whole, inasmuch as it can alternatively shift between a first deflated configuration, wherein the volume enclosed by the inner side 21 can be contracted (figure 7), and a (different) inflated configuration, wherein in the inflated configuration the volume enclosed by the inner side 21 is expanded, preferably greater than the volume enclosed by the inner side 21 in the deflated configuration.

For example, the shell 20 as a whole is able to shift from the deflated configuration to the inflated configuration by effect of the operating pressure, higher than atmospheric pressure, at which a breathable gas is supplied into the volume enclosed by the inner side 21 of the shell 20 to be made available for inhalation by a user of the mask 10, as shall become more readily apparent hereafter.

The shell 20 has at least one portion that is at least partially optically transparent.

For example, at least one portion of the flat face 24 and/or of the peripheral liner 25 is at least partially optically transparent.

In the illustrated example the whole flat face 24 is optically transparent.

In the illustrated example the whole peripheral liner 25, too, is optically transparent.

The peripheral liner 25, or its first end 251, is fastened to the flat face 24 by radio frequency welding or heat sealing.

Moreover, the peripheral liner 25 consists of a strip joined in a loop, along an (axial) junction line by radio frequency welding or heat sealing. The strip can also consist of the union of multiple strips joined together along (axial) junction lines by radio frequency welding or heat sealing.

For example, the la peripheral liner 25 has a cuff, e.g. folded inwards or outwards at its second end, distal from the flat face 24.

In practice, the end of the peripheral liner 25, at its second end, is defined by the superposition of at least two folds of the strip that constitutes the peripheral liner 25 folded back on themselves, the terminal end of the strip being positioned inside (or outside) the concavity of the shell 20.

The mask 10 comprises an annular gasket 30 fastened to the shell 20 and supported thereby.

The annular gasket 30 is for example fastened (in a permanent manner) to the shell 20 at or in proximity to the edge 23.

The annular gasket 30, in practice, surrounds the entire perimeter of the edge 23 of the shell 20.

The annular gasket 30 in fact axially prolongs the peripheral liner 25 and defines the extremal portion thereof able to go in contact with the patient's face.

In practice, the edge 23 is able to go in contact with the patient's face by interposition of the annular gasket 30.

The shell 20, as configured above, can have a weight substantially ranging between 80 and 150 grams, e.g. equal to 100 grams.

For example, the annular gasket 30 is fastened (e.g. permanently) to the second end 252 of the peripheral liner 25 of the shell 10.

For example, the annular gasket 30 is fastened to the annular liner 25 by radio frequency welding or heat sealing.

The annular gasket 30 is for example at least partially soft (yielding) and, for example, at least partially elastic/resilient.

The annular gasket 30 comprises a doughnut shaped body, for example with substantially homologous shape to that of the edge 23 of the shell 20.

The annular gasket 30 is for example an inflatable gasket, e.g. like an inflatable cushion, before the mask 10 is used.

The annular gasket 30 has an inflatable inner chamber, e.g. an air chamber.

The annular gasket 30 is thus able to shift alternatively between a first deflated configuration (figure 7) and a (different) inflated configuration, wherein in the inflated configuration the volume of the annular gasket 30 is differently, preferably greater, than the volume of the annular gasket 30 in the deflated configuration.

The annular gasket 30, for example, is defined by a first annular fold 31 e.g. flat (with substantially trapezoidal shape, as defined above for the edge 23) and by a second annular fold 32 e.g. flat (with substantially trapezoidal shape, homologous to the first annular fold 31) joined together (sealingly), e.g. exclusively, at an outer peripheral portion and/or at an inner peripheral portion.

For example, the first annular fold 31 and the second annular fold 32, at the aforesaid peripheral portions, are permanently joined together by radio frequency welding or heat sealing.

The inner chamber is defined by the volume enclosed between the first annular fold 31 and the second annular fold 32 joined at the outer peripheral portion and the inner peripheral portion of the annular folds 31 and 32.

The first annular fold 31 is for example proximal to the shell 20, e.g. to the peripheral liner 25, the second annular fold 32 is distal from the shell 20.

Each annular fold 31,32 has an inner face, oriented towards the interior of the inner chamber and an opposite outer face.

The outer face of the second annular fold 32 defines the portion of the annular gasket 30 that is more distant from the flat face 24 of the shell 20, or (at least one portion thereof) defines a bearing surface in (forced) contact with the face of the patient utilizing the mask 10 when it is worn.

The annular gasket 30, e.g. at least the outer face of the second annular fold 32 (or both annular folds), is made of an elastically yielding and/or soft plastic material, e.g. of Polyurethane (PU) or Polyvinylchloride (PVC), e.g. with high compatibility with the patient's skin.

For example, the thickness of each annular fold 31 is substantially between 0.1 mm and 0.2 mm, e.g. equal to 0.15 mm.

The annular gasket 30 is fastened (by radio frequency welding or heat sealing) to the edge 23 of the shell 20 (or of the cylindrical liner 25, e.g. at one of the two annular folds 31,32, e.g. the first annular fold 31, or at a peripheral portion of the folds (inner or outer).

The annular gasket 30, for example, comprises an inflation valve 33 through which the inner chamber is connectable with the exterior for the inflation and/or deflation thereof.

The inflation valve 33 is for example fastened to the annular gasket 30 from the opposite part relative to the bearing surface intended to bear on the patient's face.

For example, the inflation valve 33 is positioned outside the volume enclosed by the shell 20 (and by the patient's face when the mask 10 is worn by the patient).

For example, the inflation valve 33 is fastened to the first annular fold 31, e.g. in a portion thereof that is defined externally (in the radial direction) with respect to the edge 23.

The inflation valve 33 comprises a small tube fastened sealingly (e.g. by radio frequency welding or heat sealing) to the peripheral edge of a through hole drilled in one of the annular folds 31,32 and shutter means, e.g. a plug or a choking pincer (if the small tube were deformable).

For example, the inflation valve 33 can be a Schrader valve or a membrane valve or an automatic one-way valve that prevents air from spontaneously escaping from the inner chamber or the like.

For example, the annular gasket 30, as described above, weighs between 5 grams and 15 grams, preferably 10 grams.

The annular gasket 30, when in the inflated configuration, has a certain softness and adaptability to the shape of the patient's face, i.e. it has means to compensate for the play between the edge 23 of the shell 20 and the patient's face.

However, the annular gasket 30 may not be of the type with variable volume or configuration, i.e. may not be inflatable but a fixed configuration gasket, e.g. only deformable elastically and/or plastically.

The mask 10 also comprises inflow means for the inflow of a breathable gas, e.g. air or a mixture of air and oxygen or oxygen, into the volume enclosed by the shell 20 and by the annular gasket 30 (and the face of the patient, when the latter wears the mask 10).

In the example shown, the inflow means comprise an inlet conduit 40 for the injection of air.

The inlet conduit 40 (which for example has substantially rectilinear or curvilinear longitudinal axis and substantially circular cross section) is advantageously fastened, at a bound end thereof, in an irremovable manner to the mask 10, e.g. to the entire peripheral edge of a through hole drilled in the shell 20, and for example it exits in centrifugal/radial direction therefrom, homogeneously washing carbon dioxide away from the patient's mouth and nose.

The inlet conduit 40 can for example be provided, at the end that flows into the volume enclosed by the shell 20 and by the annular gasket 30 (and by the patient's face), with a diffuser.

Moreover, the inlet conduit 40 can be provided with a pressure outlet junction, e.g. branching substantially in radial direction with respect to the inlet conduit itself, to which can be associated a pressure gauge or which is closed by an appropriate plug.

The free end (distal from and external to the shell 20) of the inlet junction 40 has, for example, standard dimensions (e.g. 22M) for connection to usual instrumentations for supplying breathable gas such as air, air and oxygen or oxygen, such as tanks, conduits, flow meters, Venturi flumes or similar devices.

The inlet conduit 40 and the shell 20 constitute, in fact, a single indivisible body and, for example, are stably fastened by welding, e.g. radio frequency welding or heat sealing, or by gluing or another non-dissolvable fastening technique.

In this way, the inlet conduit 40 is in fact integrated with the mask 10, i.e. with one of its portions of which it consists.

In the illustrated example, the inlet conduit 40 is fastened to the shell 20, e.g. in any part thereof, preferably but without limitation to a portion of the peripheral liner 20, e.g. at one of the two lateral sides thereof (frontally superposed to a cheek of the patient when the latter wears the mask 10).

For example, the inlet conduit 40 is positioned in such a way that its bound end is in proximity to the patient's oro-nasal region, e.g. to the side thereof.

The mask 10 also comprises outflow means for the outflow of an exhaled gas, e.g. carbon dioxide, for escape from the interior of the volume enclosed by the shell 20 and by the annular gasket 30 (and the face of the patient, when the latter wears the mask 10) of said exhaled gas.

In the example of the figure, the outflow means comprise an outlet conduit 50 for the escape of the exhaled gas.

The outlet conduit 50 (which for example has substantially rectilinear or curvilinear longitudinal axis and substantially circular cross section) is advantageously fastened, at a bound end thereof, in an irremovable manner to the mask 10 (e.g. in a different point from the one where the inlet conduit 40 is fastened), e.g. to the entire peripheral edge of a through hole drilled in the shell 20, and for example it exits in centrifugal/radial direction therefrom.

The inlet conduit 50 can for example be provided, at the end that flows into the volume enclosed by the shell 20 and by the annular gasket 30 (and by the patient's face), with a diffuser.

Moreover, the outlet conduit 50 can be provided with a junction, e.g. branching substantially in radial direction with respect to the outlet conduit itself, to which can be associated appropriate instrumentation or which is closed by an appropriate plug.

The free end (distal from and external to the shell 20) of the outlet conduit 50 has, for example, standard dimensions (e.g. 22F or 22M) for connection to usual instrumentations for the escape of the exhaled gas, among them for example an outlet pipe, e.g. connected to a ventilator (for NIV therapy), or a PEEP valve (for CPAP therapy) and/or other similar devices.

The outlet conduit 50 and the shell 20 constitute, in fact, a single indivisible body and, for example, are stably fastened by welding, e.g. radio frequency welding or heat sealing, or by gluing or another non-dissolvable fastening technique.

In this way, the outlet conduit 50 is in fact integrated with the mask 10, i.e. with one of its portions of which it consists.

In the illustrated example, the inlet conduit 50 is fastened to the shell 20, e.g. in any part thereof, preferably but without limitation to a portion of the peripheral liner 25, e.g. at one of the two lateral sides thereof (frontally superposed to a cheek of the patient when the latter wears the mask 10), e.g. the lateral side opposite the one in which the inlet conduit 40 is positioned (e.g. substantially symmetrical relative thereto with respect to a sagittal plane of the mask 10 and of the patient).

For example, the outlet conduit 50 is positioned in such a way that its bound end is in proximity to the patient's oro-nasal region, e.g. to the side thereof.

The mask 10 can also comprise an anti-suffocation valve 60, i.e. a two-way valve able to place in communication the volume enclosed by the shell 20 and by the annular gasket (and the patient's face) with the volume outside it, in case of emergency, or when the pressure of the breathable gas inside said volume drops below a minimum threshold value, e.g. higher than (or equal to) atmospheric pressure and lower than an operating pressure useful for the NIV or CPAP therapy of the patient, which shall be better described hereafter, to whom the breathable gas is supplied through the injection means.

The anti-suffocation valve 60 can be irremovably associated to the mask 1, e.g. to any portion of the shell 20 (better if in proximity to the patient's mouth), e.g. at one portion of the flat face 24, e.g. in proximity to the lower shorter base thereof.

The anti-suffocation valve 60, as shown in figure 5, comprises a valve body which is fastened, for example irremovably, to the entire perimeter of a through hole drilled in the shell 20, e.g. in the flat face 24.

The valve body is substantially cylindrical (with reduced height), hollow, and has a bottom wall external to the shell 20, provided for example with an attachment flange fastened to the perimeter of the through hole, and an opposite bottom wall, internal to the shell 20 (to the inner volume thereof, or to its concavity).

The inner bottom wall is, for example, substantially flat and for example it lies on a parallel plane to the flat wall 24.

The inner bottom wall has a through opening, for example, a plurality of through openings defining a port for the passage of air.

The anti-suffocation valve 60 also comprises a through opening positioned on the outer bottom wall.

The outer bottom wall is, for example, substantially flat and for example it lies on a parallel plane to the flat wall 24.

In the example the through opening on the outer bottom wall (like the one obtained in the inner bottom wall) is obtained as a whole by a plurality of holes, e.g. conformed as a circular sector and concentric, which are divided from each other by radial ribs.

Advantageously, the total surface area of the through opening (on the outer bottom wall and/or on the inner bottom wall) is substantially (e.g. 5 to 20 times) greater than the passage section of a standard 22M or 22F connecting junction.

The radial ribs are joined at their central end and they join a peripheral edge of the outer bottom wall at their peripheral end.

For example, the ribs are centered on the axis of the valve body.

The outer bottom wall, at the central region where the ribs join, comprise a through central hole, e.g. substantially circular.

The anti-suffocation valve 60 also comprises a shutter positioned inside the valve body and movable from an open position of the through opening on the outer bottom wall to a closed position thereof, in contrast with thrust means.

In particular, the shutter comprises a discoidal body, which is positioned substantially parallel to the outer (and inner) bottom wall.

The discoidal body has a greater diameter than the diameter of the through opening, in order to be able to close it completely.

The discoidal body also has a smaller diameter than the inner diameter of the valve body, so that a radial interspace is defined between the periphery of the discoidal body and the inner lateral wall of the valve body.

In practice, in the closed position at least the outer peripheral edge of the discoidal body is able to come into forced contact, thanks to the action of the positive pressure inside the mask 10 (i.e. inside the volume enclosed between the shell 20, the annular gasket and the patient's face), with at least the inner peripheral edge of the outer bottom wall closing (from the interior of the valve body) substantially sealingly the through opening on the outer bottom wall, in contrast with the thrust means.

The discoidal body is for example made of at least one substantially flexible material, such as a plastic material, or alternatively it could be rigid and have a peripheral edge that projects towards the outer bottom wall made of substantially yielding material, such as rubber or plastic to achieve the seal.

The shutter comprises a central stem rising from the center of the discoidal body, which comprises one end fastened to the discoidal body itself, the free opposite end is for example provided with a widened portion, e.g. a knob.

The central segment of the central stem is for example substantially cylindrical and it is able to be inserted substantially to measure inside the central hole of the outer bottom wall, in order to slide along it between two end stop positions.

The first (closure) end stop position is defined by the contact within the valve body of the discoidal body with the outer bottom wall and defines the closed position of the shutter, the second end stop position is defined, by the contact outside the valve body, of the widened portion of the central stem with the outer bottom wall (the region where the ribs join) and defines the open position of the shutter.

In practice, the shutter is slidably associated, along the axial direction of the valve body (or otherwise in orthogonal direction to the plane of lay of the flat face 24), within the valve body, between a position approached to the bottom wall (closed position) and a position removed therefrom (open position).

The thrust means are configured to generate a thrusting force on the shutter to bring it to the open position.

The thrusting means, in the illustrated example, comprise an elastic element and preferably a spring, e.g. a compression spring, which is interposed between a discoidal body of the shutter and the outer bottom wall (in the example, the region where the ribs join, which surrounds the central hole) of the valve body.

In practice, the spring is a helical spring which is inserted on the central segment of the central stem inside the valve body.

However, the spring may be a flexure spring, a Belleville washer, or another type of spring or the thrusting means may be of a different nature, e.g. magnetic.

Optionally, the valve body may comprise, instead of the cylindrical element and of the inner bottom wall, a cross element that performs the same function, or is able to maintain the shutter in position within the valve body defining a sliding seat contained radially and axially and allowing the escape of the air that flows through the through hole drilled in the outer bottom wall.

Alternatively, the anti-suffocation valve 60 can be associated removably, as needed, e.g. to an auxiliary conduit that is wholly similar to (and distinct from) the inlet conduit 40 or the outlet conduit 50 provided in the mask 10.

The mask 10 can also comprise an additional through hole drilled in the shell 20 to whose peripheral edge is fastened a connecting element 70, e.g. of the type of a fairlead (such as SNG or the like) or another junction for various instrumentations suitable for the patient's therapy, which connect the volume outside the mask 10 with the volume enclosed by the shell 20 and by the annular gasket 30 (and by the face of the patient who wears the mask 10) and it can be closed by a membrane and/or a lid as needed.

The connecting element 70 and the shell 20 constitute, in fact, a single indivisible body and, for example, are stably fastened by welding, e.g. radio frequency welding or heat sealing, or by gluing or another non-dissolvable fastening technique.

In this way, the connecting element 70 is in fact integrated with the mask 10, i.e. with one of its portions of which it consists.

In the illustrated example the connecting element 70 is fastened to the shell 20, e.g. in any part thereof, preferably but without limitation to a portion of the peripheral liner 25, e.g. at a (lower) smaller base positioned in proximity to the chin/to the mouth of the patient or in any region of the shell 20 (of the flat face 24 or of the peripheral liner 25).

The mask 10 also comprises a plurality of fins 80 fastened to the shell 20 or to the annular gasket 30.

For example, each fin 80 has a first end, proximal to the shell 20, which is bound (irremovably or removably) to the shell 20 (or to the annular gasket 30) and an opposite second free end.

For example, the first end is fastened to the shell (or to the annular gasket 30) or is made in a single body therewith.

In the illustrated example the first end is fastened to the shell 20 at or in proximity to the edge 23.

In the example, the mask 10 comprises for example two right fins 80, e.g. separated from each other. For example, one of the two right fins 80 is an upper right fin 80, which is positioned (or extends, e.g. outwards in substantially centrifugal direction) e.g. in proximity or at a vertex to the longer base of the shell 20 (or of the edge 23), and a lower right fin 80, which is positioned (or extends, e.g. outwards in substantially centrifugal direction) e.g. in proximity or at a vertex to the shorter base of the shell 20 (or of the edge 23).

In the example, the mask 10 also comprises two left fins 80, symmetrical to the right fins 80 relative to a sagittal plane of the mask 10 (coinciding in fact with the sagittal plane of the utilizing patient).

Moreover, the mask 10 can comprise an upper fin 80, which for example is positioned (or extends, e.g. outwards in substantially centrifugal direction) e.g. in proximity to or at an intermediate point of the longer base of the shell 20 (or of the edge 23), e.g. at a median point of the longer base.

In practice, the upper fin 80 has its longitudinal axis parallel and lying on the sagittal plane of the mask 10.

Each fin 80 comprises, for example, a fastening element.

For example, a fastening element could comprise a slot or a hole or a hook obtained in the fin 80, e.g. passing through from one side thereof to the other. Alternatively and additionally, a fastening element could comprise a layer of Velcro (e.g. female) fastened (e.g. by sewing) to a side of the fin 80.

The mask 10 also comprises a nuchal strap 90, which is associated to the shell 20 (and/or to the annular gasket 30) and is able to wrap posteriorly and/or superiorly the patient's head (in the nape region of the patient) to retain the mask 10 on the face of the patient.

The nuchal strap is for example able to be fastened, e.g. removably or permanently, to the fin 80 of the mask 10.

The nuchal 90 comprises a central band 91, e.g. of rectangular shape (or any other shape), e.g. made of fabric or non-woven fabric (presenting a soft side, e.g. velveteen, intended to go in contact with the patient's nape).

The nuchal 90 comprises a plurality of arms 92, e.g. branching each from the central band 91, each of which is able to be bound (permanently or removably) to a respective fin 80.

The nuchal strap 90 in fact is able to wrap posteriorly the head (or the nape) of the patient in such a way as to retain and anchor the mask 10 solidly to the face of the patient, so that the annular gasket 30 is pulled in forced contact towards the face of the patient thereby exercising a hermetic seal for the (breathable and breathed) gases present in the internal volume of the mask enclosed by the shell 20, by the annular gasket 30 and by the face of the patient.

For example, the nuchal strap 90 has at least a left arm 92 (in the example, in the number of two) and at least one right arm 92 (in the example in the number of two) symmetrical to the respective left arm 92 with respect to a sagittal plane of the nuchal strap 20 (substantially coinciding with the sagittal plane of the mask 10 and of the patient when it is worn).

For example, each left or right arm 92 has an end that is bound to the central band 91, e.g. at or in proximity to a respective thereof, and an opposite free end.

For example, each left or right arm 92 has a longitudinal development (from the end bound to the free end) which extends away from the sagittal plane of the nuchal strap itself.

Each left or right arm 92 can be removably or permanently fastened to a respective superior or inferior, right or left fin 80.

The nuchal strap 90 could then have 2 or 4 arms 92.

Alternatively, the nuchal strap 90 could have 3 or 5 arms 92.

In this latter case the nuchal strap 90 can also comprise an upper arm 92, which for example is positioned (or extends, e.g. outwards in substantially centrifugal direction) e.g. in proximity to or at an intermediate point of an upper base of the central band 91, e.g. at a median point of the longer base.

In practice, the upper arm 92 has its longitudinal axis parallel and lying on the sagittal plane of the mask 10.

The upper arm 92 may be removably or permanently fastened to the upper fin 80.

Each arm 92 comprises, for example, a fastening element.

For example, a fastening element could comprise a button or the like able to be engaged with the hole drilled in the fin 80.

Alternatively or additionally, a fastening element could comprise a layer of Velcro (e.g. male) fastened (e.g. by sewing) to a side of the arm 92 and able to engage the (female) layer of Velcro present in a respective fin 80 or said layer of Velcro of the fin could directly engage the fabric whereof the arm 92 is made.

For example, the length of the nuchal strap 90 (or of the arms 92 and/or of the central band 91) can be adjustable.

However, a substantially rigid or elastically deformable (with shape memory) shaped ring may be associated to the mask 10, e.g. in proximity to the edge 23, e.g. in the aforesaid cuff, or to the perimeter of the flat face 24.

The mask 10 as a whole can be brought to a deflated configuration, e.g. for storage, in which it is substantially flattened.

For example, in the deflated configuration of the mask 10 the shell 20 can be in a deflated configuration thereof.

For example, in the deflated configuration of the shell 20, the shell itself presents a layout of the peripheral liner 25 that is substantially folded and parallel to the flat face 24.

For example, in the deflated configuration of the mask 10 (also) the annular gasket 30 can be in a deflated configuration thereof, in which the annular gasket 30 is substantially deflated, or flattened (with the annular folds 31 and 32 substantially superposed and contact and the inner chamber substantially empty or with reduced air content).

In practice, the maximum axial size of the mask 10 in the deflated configuration is defined by the (outer) diameter of the inlet conduit 40 and/or of the outlet conduit 50.

The shell 20 (and/or the annular gasket 30) of the mask 10 has (low) rigidity such that it can be plastically deformed, in the deflated configuration, without being able to return (completely) to an inflated configuration at atmospheric pressure, i.e. without the application of a pressure higher than atmospheric pressure into the inner volume enclosed by the shell 20 (and by the annular gasket 30 and by the patient's face).

For example, in the deflated configuration of the mask 10 the shell 20 can be in an inflated configuration thereof.

The shell 20 of the mask 10 in the inflated configuration has its maximum concavity, i.e. for example the distance between the flat face 24 and the edge 23 is the greatest. For example, the shell 20 of the mask 10 in the inflated configuration presents the peripheral liner 25 substantially parallel to the flat face 24.

The shell 20 in its inflated configuration has a surface of the inner side 21 and/or of the outer side 22 substantially smooth (tight) by effect of the positive pressure (greater than atmospheric pressure) of the gases that fill the (internal) volume enclosed by the shell 20 (and by the annular gasket 30 and by the patient's face).

For example, in the inflated configuration of the mask 10 the annular gasket 30 can be in an inflated configuration thereof.

In the inflated configuration, the annular gasket 30 has the inner chamber filled with a gas, e.g. air, at an inflation pressure greater than atmospheric pressure.

For example, the inflation pressure is variable according to use, e.g. according to the play to be compensated between the patient's face and the shell 20, i.e. its edge 23.

Inflation pressure, moreover, contributes to define the rigidity/softness of the annular gasket 30, i.e. its comfort on the patient.

The operation of the mask 10 is for example as follows.

The mask 10, for example, is initially in a deflated configuration, in which for example both the annular gasket 30 and the shell 20 are deflated.

Firstly, the annular gasket 30 can be inflated to a desired inflation pressure.

In this way, the annular gasket 30 provides a first pre-shape (substantially trapezoidal) to the mask 10 and to the edge 21 of the shell 20, such as to be able to surround a region of the patient's face, e.g. in the case of the full-face mask containing his/her eyes, nose and mouth.

Subsequently, it is possible to connect the inlet conduit 40 and the outlet conduit 50 to the respective circuits for the injection of the breathable gases and evacuation of the breathed gases and join the nuchal strap 90 to the mask 10, e.g. joining one or more of the arms 92 of the nuchal strap to one or more of the fins 80 of the mask 10.

At this point it is possible for the mask 10 to be worn on the patient's face, causing the annular gasket 30 to adhere, in forced contact, on the patient's face by effect of the pull exerted by the nuchal strap 90 on the nape of the patient.

The delivery of breathable gas at a therapeutic pressure, greater than atmospheric pressure, e.g. between 2.5 and 20 cm/H₂O, enables the shell 20 to inflate to its inflated configuration and hence to complete the shift to the inflated configuration of the mask 10 as a whole.

In addition, the delivery of breathable gas at a therapeutic pressure, greater than atmospheric pressure, makes it possible to administer an effective CPAP and NIV therapy to the patient.

The weight of the mask 10 (and its weight perceived by the patient) is decidedly lower than the rigid or semi-rigid (full-face) masks currently available on the market.

The method for the manufacture of a mask 10, e.g. an inflatable frameless full-face mask for the ventilation of patients as described above entails the following operating steps.

Initially the method can comprise cutting and shaping a first portion of a single-layer sheet of an optically transparent material, e.g. a sheet of PVC, and a second portion of a single-layer sheet of an optically transparent material, e.g. a sheet of PVC.

For example, the first portion is shaped as a flat face 24, or as a trapezoidal flat face, e.g. with rounded corners.

For example, the second portion is shaped as a (rectangular) strip of equal (or slightly greater) length to the perimeter of the flat face 24 and greater width than the maximum diameter of a connection junction 22M (or 22F), e.g. at least 3 times said maximum diameter.

For example, to the first portion can be applied an anti-suffocation valve 60 and to the second portion can be applied an inlet conduit 40, an outlet conduit 50 and a connecting element 70.

The shell 20 may be manufactured from a single portion obtained from cutting (and radio frequency welding or heat sealing) of one single-layer sheet of an optically transparent material.

The method comprises shaping as a flexible and inextensible shell 20 provided with a peripheral edge 21 closed generally as a loop able to come in forced, fluid-tight contact with the patient's face and to surround, in the example of the full-face mask shown herein, the patient's mouth, nose and eyes, e.g. starting from the aforesaid first portion and second portion.

In particular, the method comprises joining the perimeter of the first portion to one of the (long) sides of the second portion, in such a way that the second portion defines a peripheral liner 25, as described above.

The first portion is joined to the second portion by radio frequency welding or heat sealing.

The (free) side of the second portion opposite to the side joined to the first portion defines the edge 23 of the shell 20 consisting of the peripheral liner 25 and of the flat face 24.

The method also comprises obtaining an annular gasket 30, e.g. inflatable.

The annular gasket 30 is obtained, for example, cutting and shaping a first flat annular fold 31 and/or a second flat annular fold 32, starting for example from a single-layer sheet of plastic material, e.g. PU or PVC (e.g. semitransparent).

The outer or inner perimeter of each annular fold 31, 32 has the same shape and dimension as the edge 21 of the shell 20 or the inner and outer perimeter delimit an area in which it is possible to surround a line having the same shape and dimension as the edge 21 of the shell 20.

The annular gasket 30 is obtained by radio frequency welding or heat sealing only at the outer and inner peripheral portions of the annular folds 31,32, preventively superposed axially.

To one of the annular folds 31,32 is then applied an inflation valve 33.

The method then comprises joining to the edge 21 of the shell 20 at least one portion of the annular gasket 30, e.g. its inner peripheral portion, by radio frequency welding or heat sealing.

Before fastening the annular gasket 30 to the shell 20 it is possible to fasten, also by radio frequency welding or heat sealing if they are not preventively obtained in a single piece with the peripheral liner 25, the fins 80 to the edge 21 of the shell 20.

The method comprises obtaining, by cutting a layer of fabric or nonwoven fabric or another material, a nuchal strap 90 as described above for the completion of the mask 10.

The invention thus conceived can be subject to numerous modifications and variations, without departing from the scope of the invention.

In addition, all details can be replaced by other technically equivalent elements.

In practice, the materials used, as well as the contingent shapes and dimensions, may be any according to requirements without thereby departing from the scope of protection of the following claims.

## Claims

1. An inflatable frameless mask (10) for the ventilation of patients comprising:
- a flexible and inextensible shell (20) provided with a peripheral edge (23) generally closed in a loop able to come in forced, fluid-tight contact with the face of the patient and to surround at least the nose of the patient,
- inflow means (40) of a breathable gas positioned on the shell (20) for the injection of breathable gas inside a volume enclosed between the shell (20) and the face of the patient;
- outflow means (50) of gases exhaled by the patient for the exit from the volume of the gases exhaled by the patient;
- a nuchal strap (90) associated with the shell (20) for anchoring the mask (10) to the patient's head;
wherein the breathable gas introduced into the closed volume by the inflow means (40) inflates the shell (20) from a deflated configuration of the shell (20) to an inflated configuration of the shell (20), wherein in the inflated configuration the breathable gas inside the closed volume is at a higher pressure than ambient pressure; and
wherein at least one portion of the flexible shell (20) is optically transparent.

2. The mask (10) according to claim 1, wherein the peripheral edge (23) supports an annular gasket (30).

3. The mask (10) according to claim, wherein the annular gasket (30) is inflatable.

4. The mask (10) according to claim 3, comprising an inflation valve (33) on the annular gasket (30).

5. The mask (10) according to claim 1, wherein the shell (20) is completely optically transparent.

6. The mask (10) according to claim 1, wherein the shell (20) is obtained from a single layer of gas-impermeable plastic material.

7. The mask (10) according to claim 1, wherein the shell (20) comprises a flat face (24) joined to a peripheral sleeve (25) closed substantially in a loop, whose first axial end (251) is sealingly joined to the perimeter of the flat face (24) and whose second axial end is free and defines the peripheral edge (23) of the shell (20).

8. The mask (10) according to claim 1, wherein the mask (10) is full-face and the edge (23) of the shell (20) is able to inscribe the patient's mouth, nose and eyes.

9. Method for the manufacture of the inflatable frameless mask (10) according to claim 1 for the ventilation of patients comprising the following steps:
- shaping a flexible and inextensible shell (20) provided with a peripheral edge (23) generally closed in a loop able to come in forced, fluid-tight contact with the face of the patient and to surround at least the nose of the patient, wherein the shaping step comprises the step of
- joining a first shaped portion of a film of a flexible and inextensible plastic material to a second portion of a film of a flexible and inextensible plastic material by radio frequency welding or heat sealing,
- obtaining, by cutting a layer of fabric or nonwoven fabric or another material, a nuchal strap (90) for the completion of the mask (10),
- wherein at least one between the first shaped portion and the second shaped portion is at least partially optically transparent.

10. Method according to claim 9, comprising the step of joining an annular gasket (30) to the peripheral edge (23) of the shell (20) by radio frequency welding or heat sealing.

11. Method as claimed in claim 9, wherein the first shaped portion is a flat face (24) and the second shaped portion is a peripheral sleeve (25) closed substantially as a loop, whose first axial end (251) is sealingly joined, by radio frequency welding or heat sealing, to the entire perimeter of the flat face (24) and whose second axial end is free and defines the peripheral edge (23) of the shell (20).

## Patentansprüche

1. Aufblasbare rahmenlose Maske (10) zur Beatmung von Patienten, Folgendes umfassend:
- eine biegsame und nicht dehnbare Schale (20), die mit einem Umfangsrand (23) versehen ist, der im Allgemeinen in einer Schlaufe geschlossen ist, die in erzwungenen, fluiddichten Kontakt mit dem Gesicht des Patienten kommen kann und mindestens die Nase des Patienten umgeben kann,
- Einströmmittel (40) eines atembaren Gases, das an der Schale (20) für die Injektion von atembarem Gas in ein zwischen der Schale (20) und dem Gesicht des Patienten eingeschlossenes Volumen angeordnet ist;
- Ausströmmittel (50) von durch den Patienten ausgeatmeten Gasen für den Austritt aus dem Volumen der durch den Patienten ausgeatmeten Gase;
- ein Nackenband (90), das mit der Schale (20) verbunden ist, um die Maske (10) am Kopf des Patienten zu verankern;
wobei das durch das Einströmmittel (40) in das geschlossene Volumen eingeleitete atembare Gas die Schale (20) aus einer entleerten Konfiguration der Schale (20) in eine aufgeblasene Konfiguration der Schale (20) aufbläst, wobei in der aufgeblasenen Konfiguration das atembare Gas im Inneren des geschlossenen Volumens unter einem höheren Druck als dem Umgebungsdruck steht; und
wobei mindestens ein Abschnitt der biegsamen Schale (20) optisch durchlässig ist.

2. Maske (10) gemäß Anspruch 1, wobei der Umfangsrand (23) eine ringförmige Dichtung (30) trägt.

3. Maske (10) gemäß Anspruch 2, wobei die ringförmige Dichtung (30) aufblasbar ist.

4. Maske (10) gemäß Anspruch 3, umfassend ein Aufblasventil (33) an der ringförmigen Dichtung (30).

5. Maske (10) gemäß Anspruch 1, wobei die Schale (20) vollständig optisch durchlässig ist.

6. Maske (10) gemäß Anspruch 1, wobei die Schale (20) aus einer einzigen Schicht aus gasundurchlässigem Kunststoffmaterial hergestellt ist.

7. Maske (10) gemäß Anspruch 1, wobei die Schale (20) eine flache Fläche (24) umfasst, die mit einer im Wesentlichen in einer Schlaufe geschlossenen Umfangshülse (25) verbunden ist, deren erstes axiales Ende (251) abdichtend mit dem Perimeter der flachen Fläche (24) verbunden ist und deren zweites axiales Ende frei ist und den Umfangsrand (23) der Schale (20) definiert.

8. Maske (10) gemäß Anspruch 1, wobei die Maske (10) vollgesichtig ist und der Rand (23) der Schale (20) in der Lage ist, den Mund, die Nase und die Augen des Patienten zu umschließen.

9. Verfahren zur Herstellung der aufblasbaren rahmenlosen Maske (10) gemäß Anspruch 1 zur Beatmung von Patienten, folgende Schritte umfassend:
- Formen einer biegsamen und nicht dehnbaren Schale (20), die mit einem Umfangsrand (23) versehen ist, der im Allgemeinen in einer Schlaufe geschlossen ist, die in erzwungenen, fluiddichten Kontakt mit dem Gesicht des Patienten kommen kann und mindestens die Nase des Patienten umgeben kann, wobei der Formungsschritt folgenden Schritt umfasst:
- Verbinden eines ersten geformten Abschnitts eines Films aus einem biegsamen und nicht dehnbaren Kunststoffmaterial mit einem zweiten Abschnitt eines Films aus einem biegsamen und nicht dehnbaren Kunststoffmaterial durch Hochfrequenzschweißen oder Heißsiegeln,
- Erhalten eines Nackenbandes (90) zur Vervollständigung der Maske (10) durch Schneiden einer Schicht aus Stoff oder Vlies oder einem anderen Material,
- wobei mindestens einer von dem ersten geformten Abschnitt und dem zweiten geformten Abschnitt mindestens teilweise optisch durchlässig ist.

10. Verfahren gemäß Anspruch 9, umfassend den Schritt des Verbindens einer ringförmigen Dichtung (30) mit dem Umfangsrand (23) der Schale (20) durch Hochfrequenzschweißen oder Heißsiegeln.

11. Verfahren gemäß Anspruch 9, wobei der erste geformte Abschnitt eine flache Fläche (24) und der zweite geformte Abschnitt eine im Wesentlichen in einer Schlaufe geschlossene Umfangshülse (25) ist, deren erstes axiales Ende (251) durch Hochfrequenzschweißen oder Heißsiegeln mit dem gesamten Perimeter der flachen Fläche (24) abdichtend verbunden ist und deren zweites axiales Ende frei ist und den Umfangsrand (23) der Schale (20) definiert.

## Revendications

1. Masque gonflable sans cadre (10) pour la ventilation de patients comprenant :
- une coque flexible et inextensible (20) pourvue d'un bord périphérique (23) généralement fermé dans une boucle capable d'entrer en contact forcé et étanche aux fluides avec le visage du patient et pour entourer au moins le nez du patient,
- des moyens d'entrée (40) d'un gaz respirable positionné sur la coque (20) pour l'injection du gaz respirable à l'intérieur d'un volume fermé entre la coque (20) et le visage du patient ;
- des moyens d'écoulement (50) de gaz expirés par le patient pour la sortie du volume des gaz expirés par le patient ;
- une sangle nucale (90) associée à la coque (20) pour ancrer le masque (10) à la tête du patient ;
où le gaz respirable introduit dans le volume fermé par les moyens d'entrée (40) gonfle la coque (20) d'une configuration dégonflée de la coque (20) à une configuration gonflée de la coque (20), où dans la configuration gonflée le gaz respirable dans le volume fermé est à une pression supérieure par rapport à une pression ambiante ; et
où au moins une partie de la coque flexible (20) est optiquement transparente.

2. Masque (10) selon la revendication 1, où le bord périphérique (23) supporte un joint annulaire (30).

3. Masque (10) selon la revendication 2, où le joint annulaire (30) est gonflable.

4. Masque (10) selon la revendication 3, comprenant une valve de gonflage (33) sur le joint annulaire (30).

5. Masque (10) selon la revendication 1, où la coque (20) est complètement optiquement transparente.

6. Masque (10) selon la revendication 1, où la coque (20) est obtenue à partir d'une seule couche de matériau plastique imperméable au gaz.

7. Masque (10) selon la revendication 1, où la coque (20) comprend une surface plate (24) reliée à un manchon périphérique (25) sensiblement fermé en une boucle, dont la première extrémité axiale (251) est reliée de manière étanche au périmètre de la surface plate (24) et dont la seconde extrémité axiale est libre et définit le bord périphérique (23) de la coque (20).

8. Masque (10) selon la revendication 1, où le masque (10) est intégral et le bord (23) de la coque (20) est capable de marquer la bouche, le nez et les yeux du patient.

9. Procédé de fabrication du masque gonflable sans cadre (10) selon la revendication 1 pour la ventilation de patients comprenant les étapes suivantes :
- façonner une coque flexible et inextensible (20) pourvue d'un bord périphérique (23) généralement fermé dans une boucle capable d'entrer en contact forcé et étanche aux fluides avec le visage du patient et pour entourer au moins le nez du patient, où les étapes de façonnage comprennent les étapes consistant à
- relier une première partie façonnée d'une pellicule d'un matériau plastique flexible et inextensible à une seconde partie d'une pellicule d'un matériau plastique flexible et inextensible par soudage à haute fréquence ou thermosoudage,
- obtenir, en coupant une couche de tissu ou de tissu non tissé ou un autre matériau, une sangle nucale (90) pour la réalisation du masque (10),
- où au moins une entre la première partie façonnée et la seconde partie façonnée est au moins partiellement optiquement transparente.

10. Procédé selon la revendication 9, comprenant l'étape de liaison d'un joint annulaire (30) au bord périphérique (23) de la coque (20) par soudage à haute fréquence ou thermosoudage.

11. Procédé selon la revendication 9, où la première partie façonnée est une surface plate (24) et la seconde partie façonnée est un manchon périphérique (25) sensiblement fermé en une boucle, dont la première extrémité axiale (251) est reliée de manière étanche, par soudage à haute fréquence ou thermosoudage, au périmètre entier de la surface plate (24) et dont la seconde extrémité axiale est libre et définit le bord périphérique (23) de la coque (20).
